# EUROPEAN PATENT APPLICATION

(11) **EP 2 719 390 A1**
(43) Date of publication of application: **16.04.2014**
(21) Application number: 12797146.3
(22) Date of filing: 06.06.2012
(51) Int. Cl.: A61K 33/08, A61K 31/02, A61K 31/36, A61K 31/366, A61K 31/4525, A61K 33/00, A61K 33/24, A61P 1/16, A61P 3/04, A61P 3/06, A61P 9/10

(54) **CARBON-MONOXIDE(CO)-ASSISTED INHIBITION OF FATTY ACID AND CHOLESTEROL UPTAKE**

(30) Priority: 07.06.2011 JP 2011127407; 30.11.2011 JP 2011263015
(71) Applicant: Japan Science and Technology Agency, Kawaguchi-shi Saitama 332-0012 (JP)
(72) Inventor: SUEMATSU, Makoto, Tokyo 160-8582 (JP); KABE, Yasuaki, Tokyo 160-8582 (JP)
(74) Representative: Woods, Geoffrey Corlett
(86) International application number: PCT/JP2012/065058
(87) International publication number: WO 2012/169654

(57) **Abstract**

An object of the present invention is to provide a composition for inhibiting fatty acid and cholesterol uptake in a living cell which is an inhibitor for fatty acid or cholesterol uptake by a living cell, comprising carbon monoxide (CO) or a compound capable of releasing CO *in vivo.*

SEQUENCE LISTING

<110>  KEIO UNIVERSITY
       JAPAN SCIENCE AND TECHNOLOGY AGENCY

<120>  Inhibition of fatty acid and cholesterol uptake by carbon
       monoxide (CO)

<130>  N401679EP

<140>  12797146.3
<141>  2012-06-06

<150>  JP 2011-127407
<151>  2011-06-07

<150>  JP 2011-263015
<151>  2011-11-30

<160>  2

<170>  PatentIn version 3.5

<210>  1
<211>  26
<212>  RNA
<213>  Artificial

<220>
<223>  Synthetic

<400>  1
gaugugacca aaggcccgca aauucu                                          26


<210>  2
<211>  19
<212>  DNA
<213>  Artificial

<220>
<223>  Synthetic

<400>  2
gtactcagat gatgaagaa                                                  19

## Description

### Technical Field

The present invention relates to the inhibition of fatty acid and cholesterol uptake in the living cell using carbon monoxide (CO).

### Background Art

Fatty acids are the essential nutrients, particularly important as an energy source. However, when free fatty acids are excessively taken into cells such as liver cells, obesity is promoted, causing the problems of the life style-related diseases such as metabolic syndrome.

Regarding the action of carbon monoxide (CO) to the living body, it is documented that the CO production is elevated by the HO-1 (heme oxygenase-1)/CO system during the progress of atherosclerosis, causing blood vessel walls to relax (Non Patent Literature 1). Also, the same literature suggests that the system inhibits the formation of plaque in the atherosclerosis.

Further, it is documented that, in the artery exposed to CO, the permeability of endothelial membrane increases and the cholesterol uptake through the blood vessel wall increases (Non Patent Literature 2). The same literature suggests that the permeability of the endothelial membrane is a result of the widened endothelial intercellular gaps caused by CO.

Furthermore, regarding the action of CO on the blood vessels, it is documented that CO causes the vasodilation and a carbonyl compound capable of releasing CO is used for treating the hypertension or the like (Patent Literature 1).

However, there was no finding in the relation between CO and the metabolism of fatty acids, cholesterol, or the like, in a cell.

### Citation List

### Patent Literature

Patent Literature 1 JP Patent Publication (Kokai) No. 2009-215311 A

### Non Patent Literature

Non Patent Literature 1 DA-NAN LIU et al., CARDIOVASCULAR JOURNAL OF AFRICA, Vol 21, No 5, September/October 2010
Non Patent Literature 2 J.S.M. SARMA, et al., Atherosclerosis, 22(1975) 193-198

### Summary of Invention

An object of the present invention is to provide a composition for inhibiting fatty acid and cholesterol uptake in a living cell.

As described above, it was documented that the blood vessel intercellular gaps grow wider by CO, the blood vessel wall relaxes and the cholesterol uptake through the blood vessel wall increases. However, there was no finding that CO is involved with the uptake or the like of a substance by a cell.

The present inventors found that CO in a living cell inhibits the uptake of a fatty acid such as stearic acid or palmitic acid, and cholesterol. Based on this finding, the present inventors found that the conditions of metabolic syndrome or the like, with which the accumulation of a fatty acid and cholesterol in a cell is associated can be ameliorated and further the diseases related to the above conditions can be prevented or treated when CO or a compound capable of releasing *CO in vivo* is used to inhibit the uptake of a fatty acid or cholesterol in a living cell, whereby the present invention was accomplished.

More specifically, the present invention is as follows.
[1] An inhibitor for fatty acid and/or cholesterol uptake by a living cell, comprising carbon monoxide (CO).
[2] An inhibitor for fatty acid and/or cholesterol uptake by a living cell, comprising a compound capable of releasing carbon monoxide (CO) *in vivo.*
[3] The inhibitor for fatty acid and/or cholesterol uptake according to [2], wherein the compound capable of releasing carbon monoxide (CO) *in vivo* is a methylenedioxybenzene derivative compound having a methylenedioxyphenyl (MDP) group or a metal carbonyl complex.
[4] The inhibitor for fatty acid and/or cholesterol uptake according to [3], wherein the compound capable of releasing carbon monoxide (CO) *in vivo* is a methylenedioxybenzene derivative compound represented by the following formula: 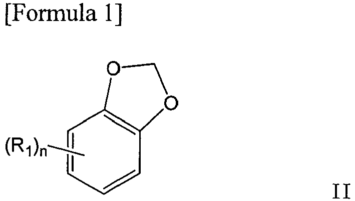 wherein R₁ represents a substituent on the benzene ring, and n represents the number of the substituent and is 0, 1, 2, 3, or 4.
[5] The inhibitor for fatty acid and/or cholesterol uptake according to [3], wherein the compound capable of releasing carbon monoxide (CO) *in vivo* is selected from the group consisting of myristicin, safrole, isosafrole, methysticin, and piperine.
[6] The inhibitor for fatty acid and/or cholesterol uptake according to [3], wherein the metal carbonyl complex capable of releasing carbon monoxide (CO) *in vivo* is a ruthenium carbonyl complex or an iron carbonyl complex.
[7] The inhibitor for fatty acid and/or cholesterol uptake according to any of [1] to [6], wherein CO inhibits fatty acid and/or cholesterol uptake by a living cell via PGRMC1.
[8] The inhibitor for fatty acid and/or cholesterol uptake according to [7], wherein CO suppresses the function of PGRMC1 by binding to a heme of PGRMC1 and fatty acid and cholesterol uptake into a cell is inhibited.
[9] An ameliorating, therapeutic, or preventive agent comprising an inhibitor according to any of [1] to [8] for a condition or a disease selected from the group consisting of metabolic syndrome, metabolic syndrome-related diseases, heart diseases or cerebrovascular diseases having an increased risk of development by metabolic syndrome, obesity, fatty liver, and non-alcoholic steatohepatitis.
[10] A body fat accumulation suppressor comprising an inhibitor according to any of [1] to [8].
[11] A method for inhibiting fatty acid and/or cholesterol uptake by a cell, comprising contacting carbon monoxide (CO) with the cell *in vitro.*

As shown in Examples, CO inhibits the uptake of a fatty acid and cholesterol in a cell. Consequently, when CO is administered to a living body or a compound capable of producing and releasing *CO in vivo,* for example a methylenedioxybenzene derivative compound having a methylenedioxyphenyl (MDP) group, is administered to a living body, the uptake of a fatty acid and cholesterol in a cell *in vivo* is inhibited and it is thus useful for treating and preventing the diseases such as metabolic syndrome with which the accumulation of a fatty acid and cholesterol in a cell is associated.

The present specification encompasses the contents described in the specification and/or drawings of Japanese Patent Application Nos. 2011-127407 and 2011-263015, which are the basis of priority of the present application.

### Brief Description of Drawings

Figure 1 is the images showing the inhibition of stearic acid uptake in cells by CO.
Figure 2 is the images showing the inhibition of LDL uptake in cells by CO.
Figure 3 is the graphs showing the absorption spectrum of the PGRMC1 protein.
Figure 4 is the images showing the inhibition of stearic acid and palmitic acid uptake in cells wherein the PGRMC1 gene is knocked down by siRNA targeting PGRMC1.
Figure 5 is a drawing showing the construct of the vector used to create the PGMRC1 knockdown.
Figure 6 is a drawing showing the presence or absence of PGRMC1 gene expression in a PGRMC1 knockdown mouse.
Figure 7 is a drawing showing the body weight gain when the PGRMC1 knockdown mouse was fed with a high fat meal.
Figure 8 is images showing the fat accumulation when the PGRMC1 knockdown mouse was fed with a high fat meal.
Figure 9 is a drawing showing the fat volume ratio when the PGRMC1 knockdown mouse was fed with a high fat meal.

### Description of Embodiments

Hereinafter, the present invention is described in detail.

The present invention is an inhibitor for fatty acid or cholesterol uptake in a living cell containing as an active ingredient carbon monoxide (CO) or a compound capable of producing CO *in vivo,* and the uptake of a fatty acid and/or cholesterol in the living cell is inhibited by causing CO to act on the cell.

The fatty acid whose uptake is inhibited by the inhibitor for fatty acid or cholesterol uptake according to the present invention is not limited but is free fatty acids including stearic acid, palmitic acid, and the like. Further, the cholesterol in the present invention encompasses cholesterols contained in the lipoprotein such as low-density lipoprotein (LDL) cholesterol, and further includes oxidized cholesterol produced when cholesterol is oxidized. Preferably, in the present invention, the cholesterols whose uptake into a cell is inhibited are those likely to cause metabolic syndrome, metabolic syndrome-related diseases, heart diseases or cerebrovascular diseases having an increased risk of development by metabolic syndrome, obesity, fatty liver such as alcoholic fatty liver, non-alcoholic steatohepatitis, or the like.

Further, a compound capable of releasing *CO in vivo* may be administered to a living body. When such a compound is administered into a living body, the compound is metabolized by the action of enzymes *in vivo* and releases CO and the uptake of a fatty acid or cholesterol in a cell of a living body is inhibited by the action of released CO. Also, the uptake of a fatty acid and cholesterol can be inhibited at the same time.

Examples of the compound capable of releasing *CO in vivo* include methylenedioxybenzene derivative compounds having the methylenedioxyphenyl (MDP) group represented by the following formula I.

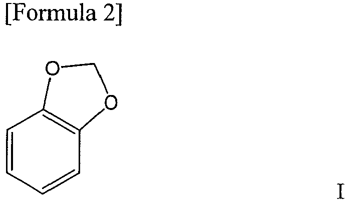

Examples of the methylenedioxybenzene derivative compound include compounds represented by the formula II. 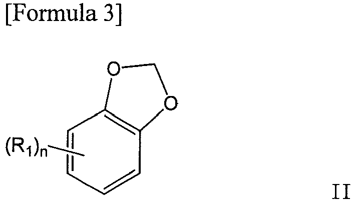 wherein R₁ represents a substituent on the benzene ring, and n represents the number of the substituent and is 0, 1, 2, 3, or 4, preferably 0 or 1.

Further, examples of the methylenedioxybenzene derivative compound include compounds represented by the formula III.

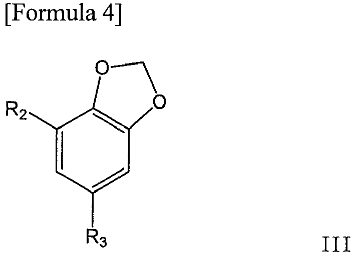

Most of these compounds are contained in plant oils and can be extracted from plants and purified. Alternatively, these compounds can be chemically synthesized.

Examples include compounds wherein R₂ is CH₃O- and R₃ is H₂C=CH-CH₂- (nutmeg (Myristica fragrans)-derived myristicin), compounds wherein R₂ is H and R₃ is H₂C=CH-CH₂- (sassafras (Sassafras albidum)-derived safrole), and compounds wherein R₂ is H and R₃ is CH₃-HC=CH- (sassafras-derived isosafrole), and further examples include birch (Betiia verrucosa)-derived methysticin represented by the following formula IV and pepper (Piper nigrum)-derived piperine represented by the following formula V.

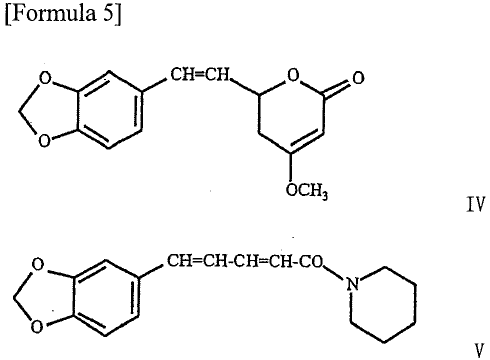

These compounds are oxidized by the enzymes belonging to the cytochrome P450 family, hydroxylase *in vivo,* as shown in the following formula, thereby producing CO. The enzymes belonging to the cytochrome P450 family are present in a large amount in the liver and CO is also mainly produced in the liver.

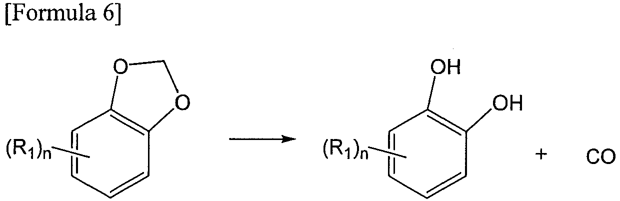

Further, metal carbonyl compounds can also be used as the compounds releasing the CO gas *in vivo.* Examples of the metal carbonyl compound include metal carbonyl complexes containing a metal and carbonyl as a ligand. Examples of the metal include ruthenium (Ru), iron (Fe), manganese (Mn), cobalt (Co), nickel (Ni), molybdenum (Mo), and rhodium (Rh), with ruthenium and iron being preferable among these.

Examples of the above metal carbonyl complex include the compounds represented by the formula [M(CO)ₐX_{b}]_{d}. In the formula, M is the above metal, X is an atom or a group binding to M by an ionic bond, covalent bond, or coordinate bond, and a, b, and c are each at least 1 wherein each X may be the same or different when b > 1. Examples of X include halogens, groups having a N, P, O, or S atom giving a lone pair of electrons to form a coordinate bond to M, or conjugated carbon groups. Examples of the compounds represented by the above formula include those represented by [Ru(CO)₃Cl₂]₂.

Further, dichloromethane (CH₂Cl₂) also releases CO by the action of cytochrome P450 *in vivo.*

When CO administered into a living body or CO released from the compound administered into a living body contacts with a cell *in vivo,* it was found that the uptake of a fatty acid or cholesterol in the cell is inhibited.

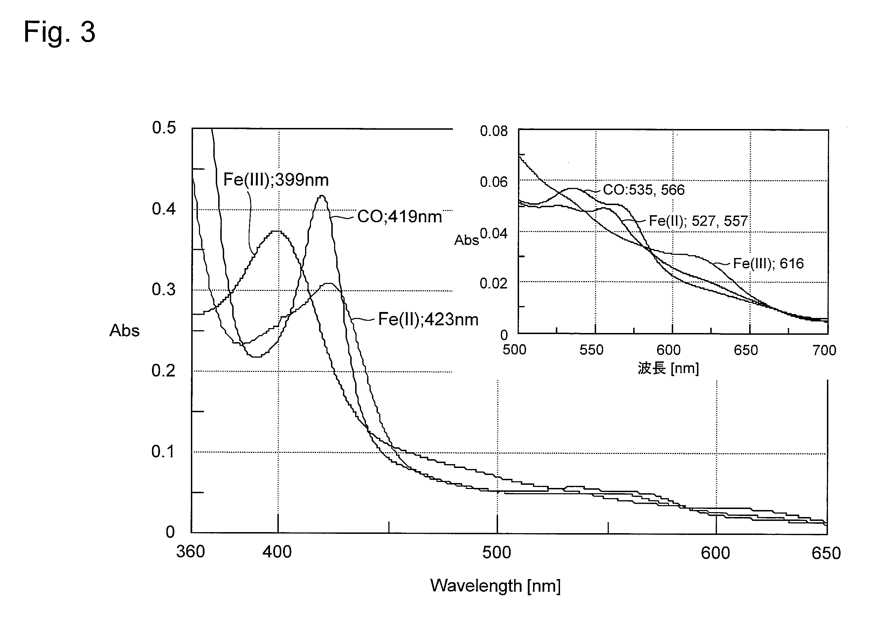

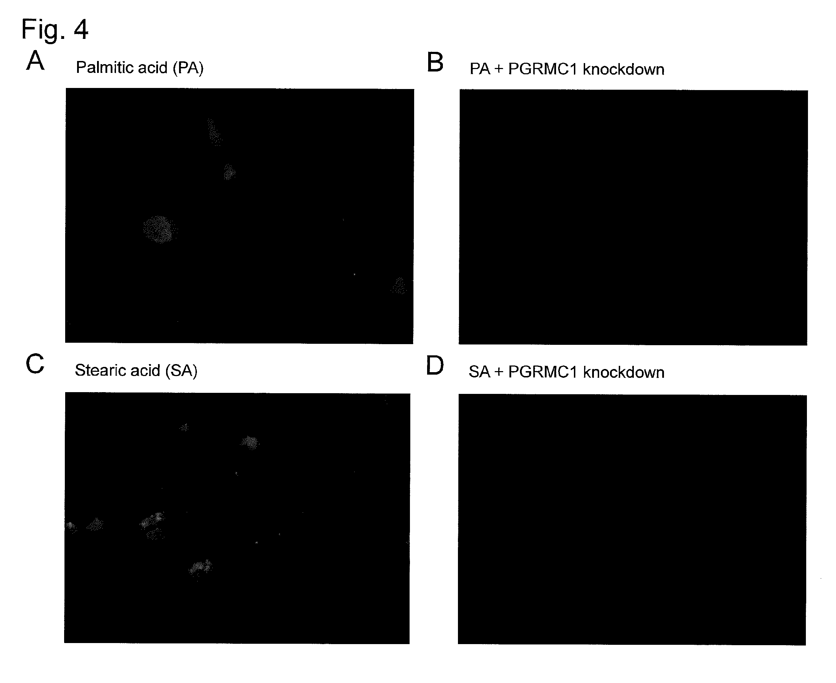

Furthermore, PGRMC1 (Progesterone Receptor Membrane associated Component 1) was identified as one of the molecules involved with the inhibition of fatty acid or cholesterol uptake by CO. PGRMC1 is a heme binding protein, and an absorption peak is thought to have appeared at 419 nm because CO bound to the heme of PGRMC1 (Figure 3). The above suggested that the function of PGRMC1 is suppressed when CO binds to the heme of PGRMC1 and the fatty acid and cholesterol uptake into a cell is inhibited. Thus, when the PGRMC1 gene was knocked down in a cell, the uptake of a fatty acid was suppressed (Figure 4).

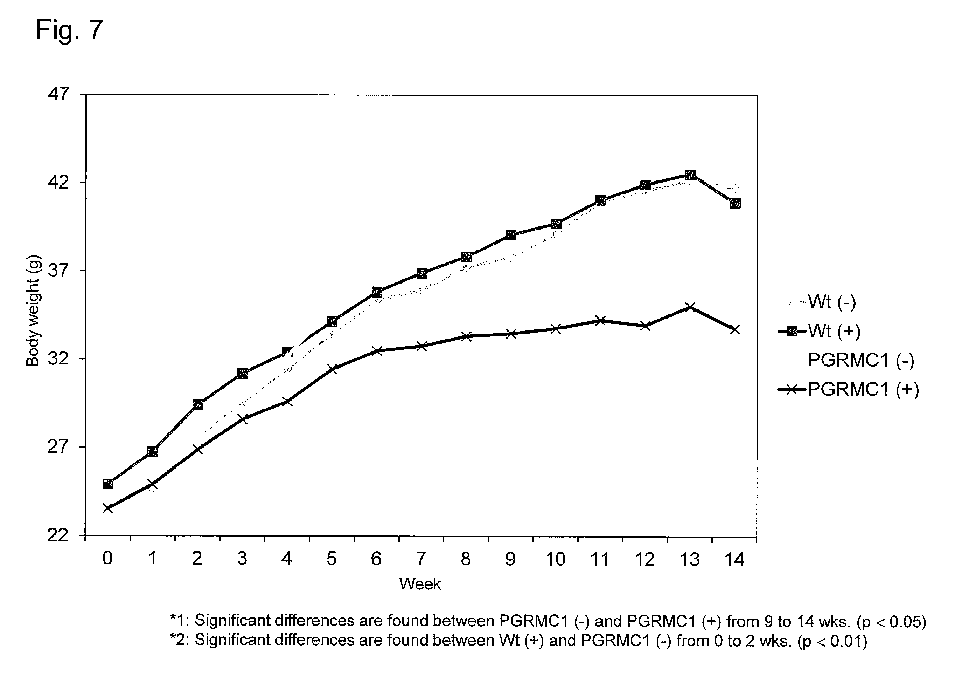

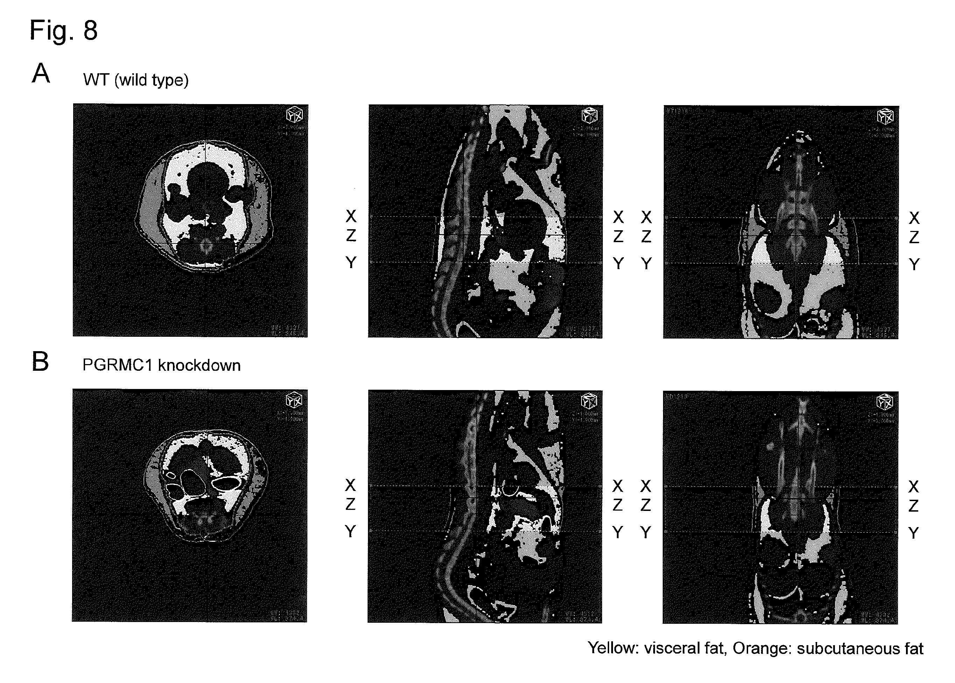

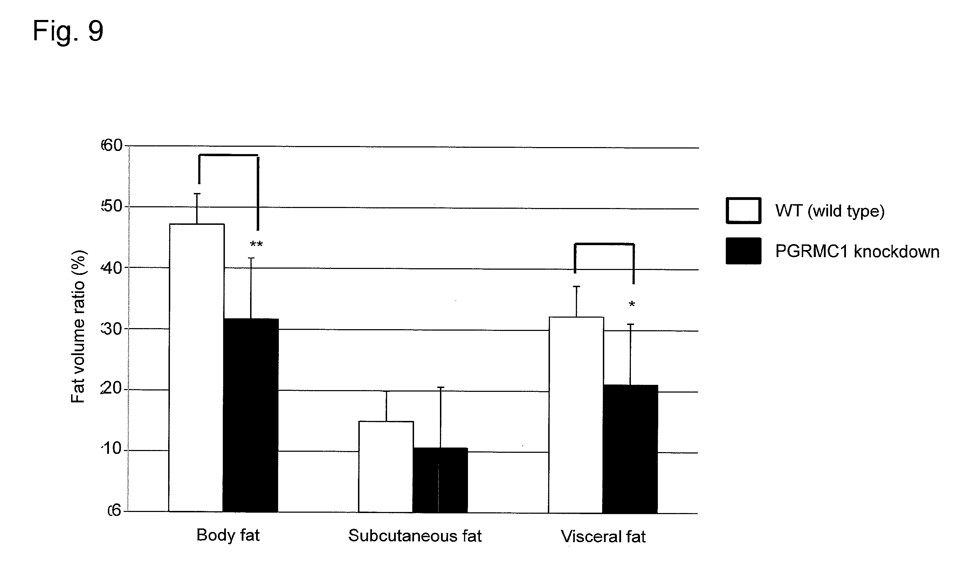

Also, in the case where a PGRMC1 knockdown mouse was fed with a high fat meal, it was found that the body weight gain was notably suppressed in comparison with the control groups (Figure 7) and the accumulation of body fat (subcutaneous fat and visceral fat) is suppressed (Figures 8 and 9).

The living body, which is targeted by the inhibitor for fatty acid or cholesterol uptake in a cell of a living body containing CO or a compound capable of releasing CO *in vivo* according to the present invention, is not limited and the living body of any animals who may store fats as the energy such as mammals, birds, or fish is the target. Of these, mammals are preferred and examples include human, cow, horse, pig, sheep, goat, cat, dog, rabbit, rat, and mouse, with human and companion animals such as dog and cat being preferable. Also, the cell to be targeted is not limited and any cells in a living body are the target. Of these, fat cells in the adipose tissue and liver cells are particularly preferred.

When a fatty acid is taken into a cell of the adipose tissue, the fatty acid binds to glycerol to form fat. The formation of fat may be a cause of metabolic syndrome, obesity, or the like.

Accordingly, when the uptake of a fatty acid and cholesterol in a cell of a living body is inhibited by CO, metabolic syndrome and obesity can be prevented or treated. The metabolic syndrome used herein refers to the condition caused by the lifestyle such as hyperlipidemia or obesity and in Japan the Ministry of Health, Labour and Welfare defines it as the case wherein a subject with a waist size of equal to or more than a predetermined size representing the visceral fat accumulation in the abdominal cavity further has two or more of lipid metabolism abnormality, hypertension, and hyperglycemia.

Additionally, the inhibitor for fatty acid or cholesterol uptake according to the present invention exhibits an ameliorating, preventive, or therapeutic effect to the conditions or metabolic diseases selected from the group consisting of metabolic syndrome, metabolic syndrome-related diseases, heart diseases or cerebrovascular diseases having an increased risk of development by metabolic syndrome, obesity, fatty liver such as alcoholic fatty liver, and non-alcoholic steatohepatitis. The diseases having an increased risk of development by metabolic syndrome refers to the disease or condition caused by metabolic syndrome and examples include hypertension, diabetes, hyperlipidemia, obesity, and diabetic complications (diabetic nephropathy, diabetic retinopathy, and the like).

The present invention encompasses a pharmaceutical composition containing an inhibitor for fatty acid or cholesterol uptake containing as an active ingredient CO or a compound capable of releasing CO *in vivo* and for ameliorating, preventing, or treating the conditions or metabolic diseases selected from the group consisting of metabolic syndrome, metabolic syndrome-related diseases, heart diseases or cerebrovascular diseases having an increased risk of development by metabolic syndrome, obesity, fatty liver such as alcoholic fatty liver, and non-alcoholic steatohepatitis. Further, the inhibitor for fatty acid or cholesterol uptake according to the present invention may also be used as, for example, a body fat accumulation suppressor.

The pharmaceutical composition of the present invention is, when CO is the active ingredient, for example, a solution containing the CO gas. In this instance, for example, a solution wherein the CO gas is dissolved may be administered by intravenous injection, intravenous drip, or the like. For the solution wherein the CO gas is dissolved, a drip transfusion such as 0.9% physiological saline, 5% glucose solution, or Ringer's solution can be used. The solution wherein the CO gas is dissolved is produced by dissolving the CO gas in an aqueous solution under applied pressure. CO can be dissolved in an amount of about 21.4 mL per liter of the aqueous solution at 25°C. The inhibitor for fatty acid or cholesterol uptake in the form of an aqueous solution according to the present invention contains, per liter of the aqueous solution, 5 mL or more, preferably 10 mL or more, further preferably 15 mL or more, particularly preferably 20 mL or more of CO. The solution wherein the CO gas is dissolved is administered to a subject by an intravenous injection, intravenous drip, or the like.

When the active ingredient is a compound capable of releasing CO *in vivo,* the form thereof is not limited and can be in the form of solid (tablet, powder, granule, or the like) or liquid (emulsion, solution, dispersion, or the like). More specifically, the pharmaceutical composition may be prepared as suspensions, emulsions, tablets, pills, capsules, chewables, aerosols, enteric coated tablets, sustained-release preparations, implant preparations, or the like.

The pharmaceutical composition may be administered via various administration routes including parenteral such as intramuscular administration, intracutaneous administration, or intravenous administration, transpulmonary, transnasal, oral, and local implantation routes.

The pharmaceutical composition may contain a carrier, diluent, and excipient, which are typically used in the field of pharmaceutical preparation. For example, lactose or the like is used as the carrier or excipient for the tablets. For the aqueous liquid for injection, physiological saline, glucose, isotonic solution containing other adjuvants, or the like is used, which may be used in combination with a suitable solubilizing adjuvant such as alcohol, polyalcohol such as propylene glycol, nonionic surfactant, or the like. For the oil solution, soybean oil or the like is used, which may be used in combination with benzyl benzoate, benzyl alcohol, or the like as the solubilizing adjuvant.

In the above pharmaceutical composition, the compound capable of releasing CO *in vivo* as the active ingredient may be contained in an amount of 0.001 to 10% by weight, preferably 0.01 to 5% by weight, further preferably 0.05 to 5% by weight.

The dose of the pharmaceutical composition of the present invention varies depending on symptoms, age, body weight, or the like, but is typically administered, in the oral administration, to an adult at 1 to 1000 mg, preferably 5 to 200 mg, in one to several divided doses per day.

CO or the compound capable of releasing CO may be, for example, locally administered to the adipose tissue or the liver.

Also, when CO or the compound capable of releasing CO *in vivo* is contacted with a cell *in vitro,* the uptake of a fatty acid and/or cholesterol by the cell can be inhibited. The present invention encompasses a method for inhibiting the uptake of a fatty acid and/or cholesterol by a cell, including contacting CO or the compound capable of releasing *CO in vivo* with the cell *in vitro.* Examples of the cell to be used include fat cells in the adipose tissue and liver cells collected from a living body. For the contact, for example, CO or the compound capable of releasing CO *in vivo* may be added to the culture medium wherein the cells are cultured. The amount of CO or the compound capable of releasing *CO in vivo* caused to contact is not limited, but for example, the compound capable of releasing CO *in vivo* may be added to the culture medium containing the cells in an amount of 0.001 to 10% by weight, preferably 0.01 to 5% by weight, further preferably 0.05 to 5% by weight. The method for inhibiting the uptake of a fatty acid and/or cholesterol by a cell *in vitro* is useful, for example, for the studies on the mechanism of the fatty acid and/or cholesterol uptake in a cell.

Further, CO or the compound capable of releasing CO *in vivo* can also be used as a reagent such as a laboratory reagent, and the present invention encompasses the reagent containing CO or the compound capable of releasing CO *in vivo.*

Furthermore, the compound capable of releasing *CO in vivo* can also be used as a drink or food composition when mixed in a drink or food product. The food product and drink include health food products, foods for specified health use, food with nutrient function claims, nutritional supplements, supplements, and the like. The food for specified health use used herein refers to food products which are ingested for the purpose of specific health maintenance in the diet and labeled to claim the achievement of the intended health maintenance by the ingestion thereof. These drink products may carry a label or the like, claiming the use thereof for ameliorating metabolic syndrome.

The food product and drink are not limited and examples include dairy products, confectioneries (biscuits, candies, jellies, ice creams, and the like), soups, juices, breads, processed meat food products (hams, sausages, and the like), noodles, processed seafood products, and seasonings (dressings and the like).

Any form may be used for livestock feed.

The present invention is described specifically with reference to the following Examples, but is not limited thereto.

### Example 1 Stearic acid uptake inhibition in a cell by CO

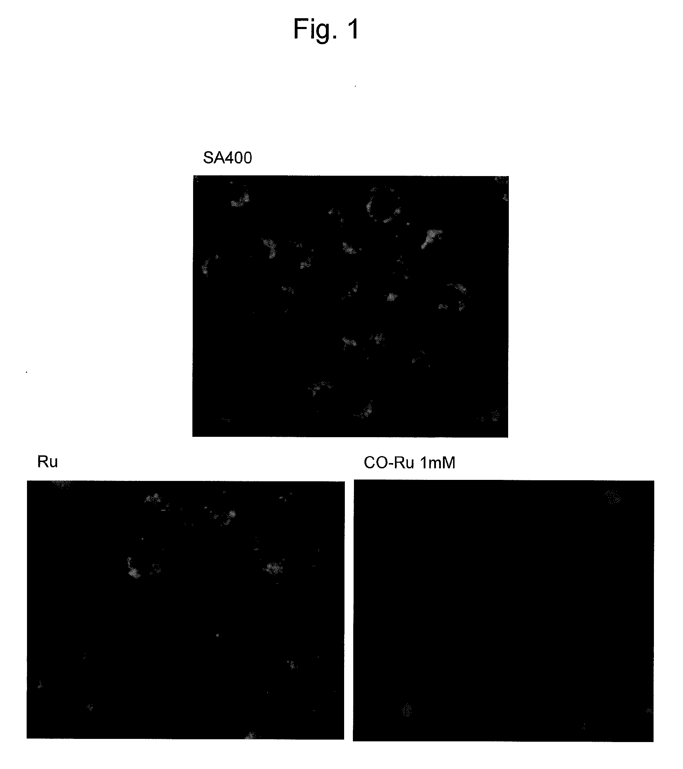

The human hepatoma cell line HuH7 cell was seeded in a 6-well plate (Iwaki) covered with a slide glass (1 cm²; matsunami glass) and cultured using DMEM + 10% FCS in a CO₂ incubator overnight. After the culture, the medium was discarded, 1% BSA (Wako Pure Chemical Industries, Ltd.) wherein stearic acid (sigma) was dissolved in serum free DMEM to give a final concentration of 400 µM was added thereto, and the cell was cultured again for 12 hours in a CO₂ incubator. Further, as a CO sustained-release drug, 1 µM of CO-Ru (Sigma CORM2 ([Ru(CO)₃Cl₂]₂)) was used) or 1 µM of ruthenium complex as a control was added together with a fatty acid to carry out the culture. Subsequently, the medium was discarded, the cell was washed 3 times with PBS, and Nile Red reagent (sigma) dissolved in PBS on the slide glass so as to be 100 ng/ml was added to react for 5 minutes, thereby staining the fatty acid. Further, the cell was washed 3 times with PBS, DAPI reagent (2 ng/ml; Wako Pure Chemical Industries, Ltd.) was added to react for 5 minutes, thereby staining the nucleus. The nucleus was observed using a fluorescence microscope, whereby Nile Red derived Green fluorescence as the fatty acid and DAPI derived Blue fluorescence were detected. The results are shown in Figure 1. In the figure, the cell to which only stearic acid was added is shown as SA400, the cell to which the ruthenium complex was added is shown as Ru, and the cell to which CO-Ru was added is shown as CO-Ru 1 µM. In the figure, the blue area shows the nucleus stained with the DAPI reagent and the green area shows the presence of stearic acid taken into the cell. In the cells to which only stearic acid was added (SA400) and the cell to which the ruthenium complex was added (Ru), the margin of blue stained area is stained green and this result suggests that stearic acid was taken into the cell. On the other hand, in the cell to which CO-Ru was added, the margin of blue stained area is not stained green. This result suggests that, in the cell to which CO-Ru was added, the uptake of stearic acid was inhibited.

### Example 2 LDL uptake inhibition in a cell by CO

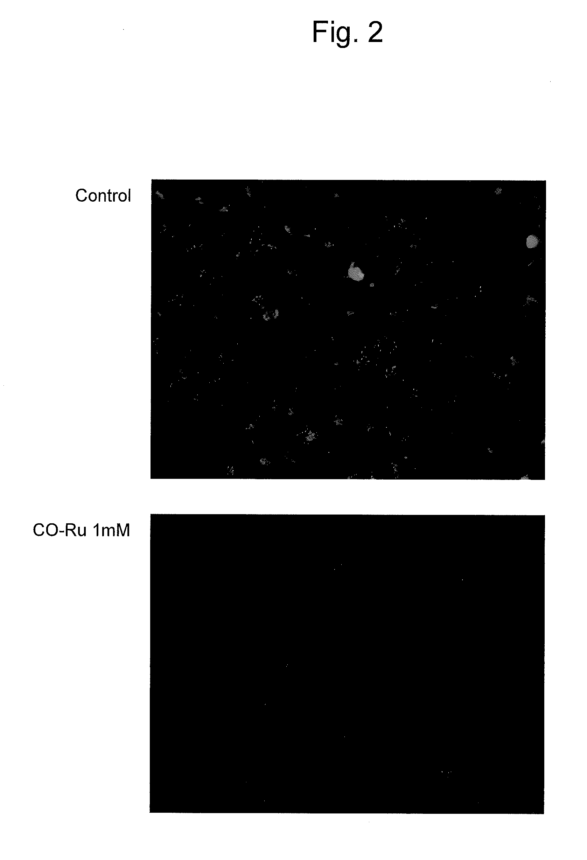

After culturing the HuH7 cell in the same manner as above in a dish covered with a slide glass, the medium was discarded, and fluorescence-labeled FITC-conjugated LDL (Molecular Probes) was added to serum free DMEM so as to give the final concentration of 8 mg/ml to carry out the culture for 2 hours (control). Additionally, at the same time with this procedure, 1 µM of CO-Ru was added in the same manner as above to carry out the analysis. After culturing, the medium was discarded, the cell was washed 3 times with PBS and DAPI reagent (2 ng/ml; Wako Pure Chemical Industries, Ltd.) was added to react for 5 minutes. The observation was carried out using a fluorescence microscope, whereby FITC-LDL derived Green fluorescence as the fatty acid and DAPI derived Blue fluorescence were detected. The results are shown in Figure 2. The top panel shows the control and the bottom panel shows the cell to which Co-Ru was added. In the control, the margin of blue stained area is stained green and this result suggests that LDL was taken into the cell. On the other hand, in the cell to which CO-Ru was added, the margin of blue stained area is not stained green. This result suggests that, in the cell to which CO-Ru was added, the uptake of LDL was inhibited.

### Example 3 Inhibition of stearic acid and palmitic acid uptake in a cell wherein the PGRMC1 gene is knocked down by siRNA targeting PGRMC1

In the experiment of Example 1, siRNA (sequence: GAUGUGACCAAAGGCCCGCAAAUUCU) (SEQ ID NO: 1) targeting PGRMC1 was transfected to the cell line HuH7 cell derived from human hepatocellular carcinoma by the lipofection method (Lipofectamin 2000, INVITROGEN Corporation) to knockdown the PGMRMC1 gene. In the same manner as in Example 1, 1% BSA (Wako Pure Chemical Industries, Ltd.) wherein stearic acid (sigma) or palmitic acid was dissolved to give the final concentration of 400 µM was added to the HuH7 cell wherein the PGRMC1 gene was knocked down, and the cell was cultured. The medium was discarded, the cell was washed 3 times with PBS, and Nile Red reagent (sigma) dissolved in PBS on the slide glass so as to be 100 ng/ml was added to react for 5 minutes, thereby staining the fatty acid. Further, the cell was washed 3 times with PBS, DAPI reagent (2 ng/ml; Wako Pure Chemical Industries, Ltd.) was added to react for 5 minutes, thereby staining the nucleus. The nucleus was observed using a fluorescence microscope, whereby Nile Red derived Green fluorescence as the fatty acid and DAPI derived Blue fluorescence were detected. The results are shown in Figure 4. In the figure, the blue area shows the nucleus stained with the DAPI reagent and the green area shows the presence of stearic acid or palmitic acid taken into the cell. When palmitic acid (Figure 4A) or stearic acid (Figure 4B) was added to the cell wherein the PGRMC1 gene was not knocked down, the margin of blue stained area is stained green and this result suggests that stearic acid or palmitic acid was taken into the cell. On the other hand, when palmitic acid (Figure 4C) or stearic acid (Figure 4D) was added to the cell wherein the PGRMC1 gene was knocked down, the margin of blue stained area is not stained green. This result suggests that, in the PGRMC1 knockdown cell, the uptake of stearic acid and palmitic acid was inhibited.

### Example 4 Effect on fat accumulation caused by high fat meal administration using PGRMC1 knockdown mouse (PGRMC1 KD mouse)

### (1) Creation of PGRMC1 knockdown mouse

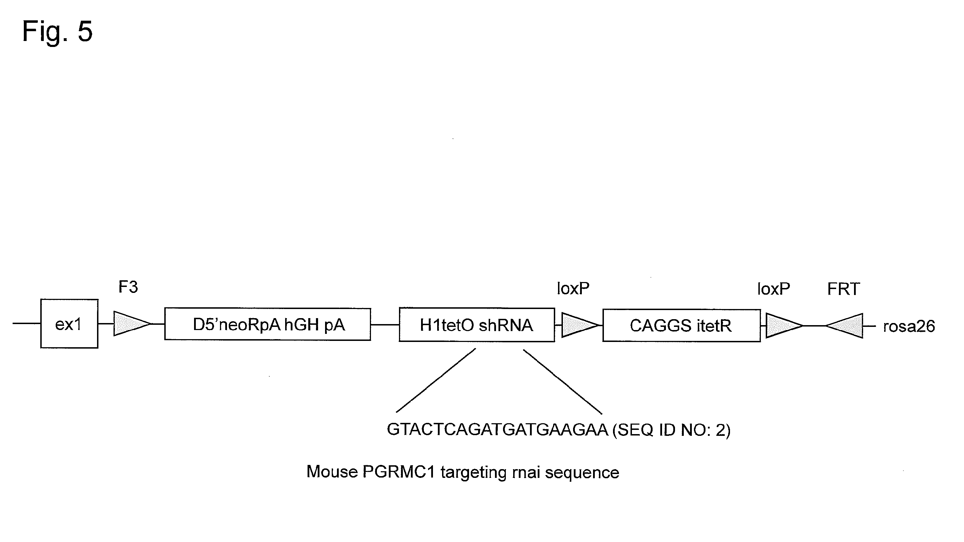

A mouse with the doxycycline-dependent PGRMC1 gene knockdown was created. The mouse strain used was C57/BL6, and the construct shown in Figure 5 was introduced to the mouse by a known method for the creation. In such a mouse, H1 promoter regulated by tetR was inserted at the upper stream of the shRNA gene wherein shRNA does not express in the absence of doxycycline administration, but shRNA expresses when doxycycline is administered to the mouse, whereby the PGRMC1 expression is suppressed in the mouse. Thus, the mouse in which the doxycycline-induced PGRMC1 expression is suppressed is termed as the PGRMC1 knockdown mouse.

### (2) PGRMC1 expression in the PGRMC1 knockdown mouse

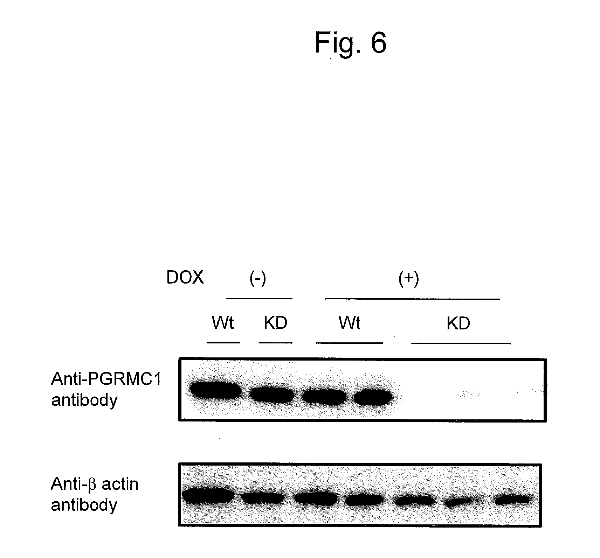

After giving water containing 2 g/L of doxycycline (DOX) as drinking water to a wild type mouse and the PGRMC1 knockdown mouse (male, 8 weeks of age), the PGRMC1 expression in the liver was examined by the western blot method using an anti-PGRMC1 antibody. As shown in Figure 6, when doxycycline was given to the PGRMC1 knockdown mouse, it was verified that the PGRMC1 expression was suppressed.

### (3) Body weight gain of the PGRMC1 knockdown mouse

Drinking water with or without doxycycline was given to the PGRMC1 knockdown mouse and the wild type mouse, high fat meals (Fat: 40%, Sucrose: 40%, Research Diets, Inc. D12327) were given, and the body weight was measured over a period of 3.5 months (14 weeks).

The results are shown in Figure 7. Wt (-) and Wt (+) in the figure show the change in body weight of the wild type mouse when doxycycline was not or was given, respectively. Also, PGRMC1 (-) and PGRMC1 (+) show the change in body weight of the PGRMC1 knockdown mouse when doxycycline was not or was given, respectively. As shown in Figure 7, the PGRMC1 knockdown mouse to which doxycycline was given had the notably suppressed body weight gain when compared with the control group.

### (4) Fat accumulation in the PGRMC1 knockdown mouse

The Wt (+) and PGRMC1 (+) mice used in the above (3) were analyzed for the accumulations of visceral fat and subcutaneous fat by the CT scan on week 14. The results are shown in Figure 9. Figure 8A shows the CT scan image of wild type mouse and Figure 8B shows the CT scan image of the PGRMC1 knockdown mouse. The left images of Figure 8A and Figure 8B show the cross sectional views, the middle images of Figure 8A and Figure 8B show the side views of mouse's fat accumulations obtained by processing the CT scan data, and the right images of Figure 8A and Figure 8B show the top views of mouse's fat accumulations obtained by processing the CT scan data. The cross sectional views along the line Z to Z in the middle images and right images are the cross sectional views of the left images. In the results of CT scan analysis, the visceral fat area is shown in yellow and the subcutaneous fat area is shown in orange. In the figures, in the left images, the white areas found in the central part of the mouse's body show the visceral fat. The gray areas therearound show the subcutaneous fat. In the middle and right images, in the region between X to X and Y to Y the white areas show the visceral fat and the gray areas therearound show the subcutaneous fat. As shown in the figures, the fat accumulation was suppressed in the PGRMC1 knockdown mouse in which the PGRMC1 gene expression was suppressed. Further, Figure 9 shows the volume ratio of fat (the percentage of the fat volume to the body volume) accumulated in the wild type mouse and the PGRMC1 knockdown mouse at week 14. The body fat is the sum of the subcutaneous fat and visceral fat. As shown in Figure 9, the PGRMC1 knockdown mouse had a reduced fat volume ratio in comparison with the wild type mouse.

From the above results, it was suggested that PGRMC1 possibly regulates the fat metabolism in a living body.

### Industrial Applicability

The inhibitor of fatty acid and/or cholesterol uptake by a cell, containing CO or a compound capable of releasing *CO in vivo* according to the present invention can be used as a pharmaceutical product or food product.

All publications, patents, and patent applications cited herein shall be incorporated per se by references in the present specification.

## Claims

1. An inhibitor for fatty acid and/or cholesterol uptake by a living cell, comprising carbon monoxide (CO).

2. An inhibitor for fatty acid and/or cholesterol uptake by a living cell, comprising a compound capable of releasing carbon monoxide (CO) *in vivo.*

3. The inhibitor for fatty acid and/or cholesterol uptake according to claim 2, wherein the compound capable of releasing carbon monoxide (CO) *in vivo* is a methylenedioxybenzene derivative compound having a methylenedioxyphenyl (MDP) group or a metal carbonyl complex.

4. The inhibitor for fatty acid and/or cholesterol uptake according to claim 3, wherein the compound capable of releasing carbon monoxide (CO) *in vivo* is a methylenedioxybenzene derivative compound represented by the following formula: 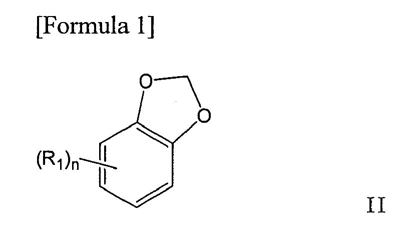 wherein R₁ represents a substituent on the benzene ring, and n represents the number of the substituent and is 0, 1, 2, 3, or 4.

5. The inhibitor for fatty acid and/or cholesterol uptake according to claim 3, wherein the compound capable of releasing carbon monoxide (CO) *in vivo* is selected from the group consisting of myristicin, safrole, isosafrole, methysticin, and piperine.

6. The inhibitor for fatty acid and/or cholesterol uptake according to claim 3, wherein the metal carbonyl complex capable of releasing carbon monoxide (CO) *in vivo* is a ruthenium carbonyl complex or an iron carbonyl complex.

7. The inhibitor for fatty acid and/or cholesterol uptake according to any one of claims 1 to 6, wherein CO inhibits fatty acid and/or cholesterol uptake by a living cell via PGRMC1.

8. The inhibitor for fatty acid and/or cholesterol uptake according to claim 7, wherein CO suppresses the function of PGRMC1 by binding to a heme of PGRMC1 and fatty acid and cholesterol uptake into a cell is inhibited.

9. An ameliorating, therapeutic, or preventive agent comprising an inhibitor according to any one of claims 1 to 8 for a condition or a disease selected from the group consisting of metabolic syndrome, metabolic syndrome-related diseases, heart diseases or cerebrovascular diseases having an increased risk of development by metabolic syndrome, obesity, fatty liver, and non-alcoholic steatohepatitis.

10. A body fat accumulation suppressor comprising an inhibitor according to any one of claims 1 to 8.

11. A method for inhibiting fatty acid and/or cholesterol uptake by a cell, comprising contacting carbon monoxide (CO) with the cell *in vitro.*
